# EUROPEAN PATENT APPLICATION

(11) **EP 3 618 002 A1**
(43) Date of publication of application: **04.03.2020**
(21) Application number: 18191730.3
(22) Date of filing: 30.08.2018
(51) Int. Cl.: G06T 11/60, G06T 7/00, A61B 5/00, A61B 8/12, A61B 8/00

(54) **INTERACTIVE SELF-IMPROVING ANNOTATION SYSTEM FOR HIGH-RISK PLAQUE BURDEN ASSESSMENT**

(71) Applicant: Koninklijke Philips N.V., 5656 AE Eindhoven (NL)
(72) Inventor: NICKISCH, Hannes, 5656 AE Eindhoven (NL); WISSEL, Tobias, 5656 AE Eindhoven (NL); GRASS, Michael, 5656 AE Eindhoven (NL)
(74) Representative: de Haan, Poul Erik

(57) **Abstract**

A medical image annotation system for analyzing a medical image. A plurality of image annotation tools are provided by the image annotation system, each of which is configured to perform, for one or more regions of interest of the medical image, at least a portion of an annotation. A recording module of the image annotation system is configured to record, for each of the regions of interest, interactions which are performed using the image annotation tools. The image annotation system is configured to compute an image annotation complexity metric for each of the regions of interest, based on the recorded interactions. Further, a presentation of the annotation tools by the user interface is indicative of an order, wherein the order is changed in response to the region of interest from which the user input is currently received.

## Description

### FIELD OF THE INVENTION

The invention relates to a system and method for interactive annotation of medical images.

### BACKGROUND OF THE INVENTION

Coronary artery disease (CAD) is one of the largest causes of death worldwide. Suspicious narrowings in the coronaries need to be accurately delineated on a lesion level by the physician in order to allow for a comprehensive and quantitative risk assessment followed by a conclusive treatment decision.

Different patterns such as calcification levels as well as so-called high-risk plaque features need to be located and segmented accurately to enable a reliable assessment of the overall plaque burden. Intravascular imaging modalities such as intra vascular ultrasound, IVUS, and optical coherence tomography, OCT as well as organ-level imaging modalities such as ultrasound, US, magnetic resonance imaging, MRI, and computed tomography, CT, have complementary properties and are typically used to provide the anatomical and functional information required to make predictive statements based on the plaque composition.

In the context of medical image annotation, computer systems are somewhat complementary to human operators. They excel at repetitive tasks demanding constant attention i.e. counting cells or collecting and quantifying local evidence but show shortcomings when contextual information or global prior knowledge is available.

The following documents are known in the field of the present disclosure and in related fields:
[1] João Silva Marques and Fausto J. Pinto, The vulnerable plaque: Current concepts and future perspectives on coronary morphology, composition and wall stress imaging, Rev Port Cardiol. 33(2):101-110, 2014.
[2] Michiel J. Bom, Dirk J. van der Heijden, Elvin Kedhi, Jan van der Heyden, Martijn Meuwissen, Paul Knaapen, Stefan A.J. Timmer, Niels van Royen, Early Detection and Treatment of the Vulnerable Coronary Plaque, Can We Prevent Acute Coronary Syndromes? Circ Cardiovasc Imaging. 2017;10:e005973.

Because the image data typically used in such procedures is high-dimensional and potentially of very high-resolution, the annotation of these datasets is desirably at least partly automated since a complete manual annotation at a voxel level may be too time-consuming to fit in a clinical workflow. Here, annotation software may thus play an important role. Elementary operations for efficient 2D segmentation - beyond mere voxel-level annotation - may include for example Interactive Level Sets, Brush Strokes, Spline Curves and Bounding Boxes. These are just examples, and many more editing tools are of course possible. A particular challenge is the 3D nature of most medical images which conflicts with the available editing and visualization tools that are most suited for 2D data. Fully automatic annotation algorithms (possibly learned from previously recorded human annotations) form the other end of the spectrum, as they do not require user interaction at all. Very often, hybrid algorithms are employed in practice forming a so-called semi-automatic annotation tool, where the software proposes an annotation, which is later accepted, refined, improved, corrected or simply rejected by the human operator.

However, current state of the art annotation tools (e.g. coronary centerline, lumen and wall editing) are based on automatic annotation and smoothing algorithms that are optimized to automatically solve the annotation task up to a certain level of accuracy. These algorithms are not optimized to accomplish the annotation task with minimal user interaction. The invention seeks to address one or more of the aforementioned drawbacks.

### SUMMARY OF THE INVENTION

The present disclosure provides a method of minimizing the required user interaction effort for annotating medical images. Specifically, the present disclosure allows accurate delineation of coronary plaques, the lumen boundary and/ or the media-adventitia border. Delineation of coronary plaque may be used in the risk assessment for future acute coronary syndrome, ACS. A segmentation system may be configured to use a predictive forward user model trained on the logs of previous semi-automatic segmentation sessions. Thereby, the segmentation system may assist a user in such a way that the overall time required for a complete segmentation is minimized.

Embodiments of the present disclosure pertain to a system comprising a medical image annotation system for analyzing a plurality of two- and/ or three-dimensional medical images. The medical image annotation system provides a plurality of image annotation tools, each of which being configured to perform, for one or more regions of interest of the medical image, at least a portion of an annotation. The medical image annotation system comprises a user interface, a recording module and a computation module. For each of the images, the user interface is configured to i) present the respective medical image, and to ii) receive, for each of the regions of interest of the respective image, user input corresponding to one or more interactions using one or more of the image annotation tools. The recording module is configured to record for one or more of the regions of interest of at least a first one of the images each interaction. At least one of (a) and (b) applies for the first image and/ or a second image of the images: (a) the computation module is configured to compute an image annotation complexity metric for each of the regions of interest of the respective image, depending on the recorded plurality of interactions; and (b) a presentation of the annotation tools by the user interface is indicative of an order, wherein the order is changed in response to the region of interest of the respective image from which the user input is currently received.

The analysis of the medical image may include annotating one or more regions of interest within the medical image. The location of the regions of interest within the medical image may change during the annotation. By way of example, during the annotation, the identification of the pixels and/ or voxels which form part of the region of interest may be refined. The term "annotation" may be defined herein to mean that one or more pixels of the medical image is assigned to one or more predefined classes. The classes may be classes of a body portion and/ or classes of borders between different body portions such as a lumen border of a blood vessel or a media-adventitia border of a blood vessel. The annotation may include determining an extent of the region of interest within the medical image. The region of interest may correspond to an image structure of the image. By way of example, the image structure may represent a lumen border or a media-adventitia border of a blood vessel. The annotations which are performed for the medical image may result in a segmentation of the medical image into the plurality of regions of interest. The regions of interest may be overlapping or non-overlapping.

The images may have a common set of regions of interest. By way of example, each of the images may have a region of interest for the lumen border and a further region of interest for the media-adventitia border. In each of the images, the region of interest may be located at a different location within the image.

Examples for regions of interest are but are not limited to: at least a portion of a plaque or of a boundary of plaque in a blood vessel, at least a portion of a lumen or of a lumen border of a blood vessel, at least a portion of an extent of a blood vessel or of a media-adventitia border of the blood vessel. The blood vessel may be a coronary blood vessel.

At least a portion of the annotation tools may be semi-automatic, i.e. requiring the user interaction.

The medical image annotation system may include a data processing system. The data processing system may include a computer system having a processor and a memory for storing instructions processable by the processor. The processor may execute an operating system. The processor may perform operations to perform the method steps and operations discussed within the present disclosure. The data processing system may further include the user interface of the image annotation system. The user interface may be configured to allow a user to receive data from the data processing system and/ or and/ or to provide data to the data processing system. The user input may be received via input devices of the data processing system, such as a computer mouse and/ or and/ or a keyboard. The user interface may include a graphical user interface. The data processing system may further include a display device for presenting to the user the medical image and the image annotation tools using the user interface. The medical images may be acquired using one or a combination of the following techniques: angiography (such as coronary CT angiography, abbreviated as cCTA), angioscopy, thermography, fluorescence microscopy, intravascular ultrasound (IVUS), optical coherence tomography (OCT), computer tomography (CT), magnetic resonance imaging (MRI), positron emission tomography (PET), and/ or single-photon emission computed tomography (SPECT). The medical image may be acquired using microscopic image acquisition.

Each of the medical images may include greyscale image data and/ or color image data. Each of the medical images may show one or more image structures, each of which representing one or more anatomically and/ or functionally defined portions of the body. Each of the regions of interest may correspond to at least a portion of an image structure and/ or to at least a portion of a border of the image structure. Examples for an anatomically defined portion of the body are tissue structures, such as blood vessels. A functionally defined portion of the body may be a portion of the body, which performs an anatomical function, such as plaque which is in a blood vessel. At least one of the images may show a cross-section through a blood vessel. The cross-section maybe substantially perpendicular or substantially parallel to a longitudinal axis of the blood vessel.

The one or more interactions for the region of interest may be performed for annotating the respective region of interest. Each of the interactions may include applying one or a combination of image annotation tools to the medical image.

The image annotation complexity metric may include one or more parameters. The computed image annotation complexity metric maybe stored in a storage device of the image annotation system.

At least a portion of the annotation tools may be presented to the user using a graphical representation for each of the annotation tools. The graphical representation may be an icon. The term "image annotation tool" may be defined herein to mean one or more operations (in particular numerical operations), which are applied to one or more pixels and/ or voxels of the medical image. The pixels and/ or voxels at least in part be selected by the user. One or more of the image annotation tools may be configured to identify one or more pixels and/ or one or more voxels of the medical image in order to define an extent of the region of interest. The order of the image annotation tools may be a hierarchical order. In other words, the image annotation tools may be presented in a graded order so that the order of an image annotation tool reflects a rank of the image annotation tool among the remaining image annotation tools. The order may be an order of preference for performing an efficient image annotation requiring a low degree of user interaction. By way of example, the annotation tools may be presented in a geometric arrangement on a display device of the system, wherein the geometrical arrangement is indicative of the order. Additionally or alternatively, one or more numbers maybe assigned to one or more annotation tools and displayed concurrently with the respective annotation tools, wherein the numbers are indicative of the order of the annotation tools.

According to an embodiment, wherein at least (a) applies and for at least one of the regions of interest of the respective image, the image annotation complexity metric of the region of interest is determined depending one or more of the interactions recorded for the respective region of interest.

According to a further embodiment, at least (a) applies and for at least one of the regions of interest of the respective image, the image annotation complexity metric is indicative of a degree of user interaction and/ or an amount of user interaction required to annotate at least a portion of the region of interest using the user interface. The degree of user interaction may be a measure of the relative amount of operations performed by the user to all operations required for annotating at least the portion of the region of interest.

According to a further embodiment, at least (a) applies and for at least one of the regions of interest of the respective image, the image annotation complexity metric is determined depending on a measured time required by the user to annotate at least a portion of the region of interest via the user interface.

According to a further embodiment, at least (a) applies and for at least one of the regions of interest of the respective image, the image annotation complexity metric is determined depending on a number of interactions required by the user to annotate at least a portion of the region of interest via the user interface.

According to a further embodiment, at least (a) applies and for at least one of the regions of interest of the respective image, the image annotation complexity metric is determined depending on which of the plurality of annotation tools are used by the user to annotate at least a portion of the region of interest.

According to a further embodiment, at least (a) applies and for at least one of the regions of interest of the respective image, the image annotation complexity metric is determined depending on a number and/ or depending a geometrical arrangement of the pixels and/ or voxels of at least a portion of the region of interest. By way of example, the image annotation complexity metric may be determined depending on one or more parameters which are determined depending on the geometrical arrangement, such as depending on a pixel cluster size distribution. By way of example, the parameters determined depending on the pixel cluster size distribution may include a mean cluster size and/ or a number of pixel clusters below a predefined threshold cluster size.

According to a further embodiment, (a) and (b) applies and the image annotation system is configured to determine, for at least one of the regions of interest of the respective image, the order of the image annotation tools depending on the image annotation complexity metric of the region of interest.

According to a further embodiment, the user interface is configured to adapt one or more operation parameters of one or more of the image annotation tools depending on one or more of the recorded interactions. An operation parameter may be a parameter on which the extent of the region of interest depends if the annotation tool us used to perform the annotation. The extent of the region of interest may be represented by the group of pixels which form the region of interest.

According to a further embodiment, the system is configured to vary the at least one of the operational parameters. The system may further be configured to measure, how the variation influences the measurements acquired from at least a portion of the interactions of the user. The measurements acquired from the interactions may include a measurement of a time required by the user for performing the interactions and/ or a number of the interactions.

According to a further embodiment, at least (a) applies and the user interface is configured to display, for at least one of the regions of interest of the respective image, an indicator which is visually indicative of the image annotation complexity metric of the region of interest and which is displayed concurrently with the region of interest. The indicator and the medical image may be displayed by the user interface in an overlaid manner. The indicator may be visually indicative of the extent of at least a portion of the region of interest.

According to a further embodiment, at least (b) applies and the image annotation system is configured to generate a user profile depending on the user input received via the user interface, wherein the order of the annotation tools is determined depending on the user profile. The user profile may be indicative of a classification of the user into a plurality of pre-defined user classes. The classes may be classes of user experience. By way of example, the user classes may include the classes "experienced user" and "unexperienced user".

The medical image annotation system may be configured to receive user input indicative of a user identifier of a user who performs the annotation of the one or more regions of interest. The medical image annotation system may be configured to store the user profile on a storage device of the medical image annotation system. The medical image annotation system may be configured to determine the image annotation complexity metric and/ or the order of the image annotation tools depending on one or more parameters of the user profile, in particular depending on the classification of the user.

Embodiments of the present disclosure pertain to a method of analyzing a plurality of two- and/ or three-dimensional medical images using an image annotation system having a user interface. The medical image annotation system provides a plurality of image annotation tools, each of which being configured to perform, for one or more regions of interest of the medical images, at least a portion of an annotation. The method comprises for each of the images: presenting, using the user interface, the respective medical image; receiving, using the user interface, for each of the regions of interest of the respective image, user input corresponding to one or more interactions using one or more of the image annotation tools. For a first one of the images, the method comprises recording, using the image annotation system, for one or more of the regions of interest of the first image, the interactions. At least one of (a) and (b) applies for the first image and/ or a second image of the images: (a) the method further comprises computing an image annotation complexity metric for each of the regions of interest of the respective image, depending on the recorded interactions; and (b) the method further comprises presenting the annotation tools using the user interface, so that the presentation is indicative of an order, wherein the order is changed in response to the region of interest of the respective medical image from which the user input is currently received.

Embodiments of the present disclosure relate to a computer program product which comprises instructions which when executed on a computer cause the computer to carry out the method steps described herein.

Embodiments of the present disclosure pertain to a program element for analyzing a plurality of two-dimensional and/ or three-dimensional medical images using an image annotation system having a user interface. The medical image annotation system provides a plurality of image annotation tools, each of which being configured to perform, for one or more regions of interest of the medical image, at least a portion an annotation. The program element, when being executed by a processor of the data processing system, is adapted to carry out for each of the images: presenting, using the user interface, the respective medical image; receiving, using the user interface, for each of the regions of interest of the respective image, user input corresponding to one or more interactions using one or more of the image annotation tools. The program element, when being executed by a processor of the data processing system, is further adapted to carry out for a first one of the images: recording, using the image annotation system, for one or more of the regions of interest of the first image, the interactions. At least one of (a) and (b) applies for the first image and/ or a second image of the images: (a) the program element, when being executed by the processor, is adapted to carry out: computing an image annotation complexity metric for each of the regions of interest of the respective medical image, depending on the recorded interactions; and (b) the program element, when being executed by the processor, is adapted to carry out: presenting the annotation tools by the user interface so that the presentation is indicative of an order, wherein the order is changed in response to the region of interest of the respective image from which the user input is currently received.

Embodiments of the present disclosure pertain to a medical image annotation system for use in delineating coronary plaque in medical images. The system comprises a user interface for presenting medical images and a plurality of image annotation tools to a user; a recording module, a computation module, and an output module. The user interface is configured to i) present a medical image and a plurality of image annotation tools having a hierarchical order, the medical image including a plurality of regions of interest, and to ii) receive user input corresponding to one or more interactions with each image annotation tool for each region of interest of the medical image. The recording module is configured to record each interaction with each image annotation tool for each region of interest of the medical image. The computation module is configured to compute an image annotation complexity metric for each region of interest of the medical image, based on the recorded plurality of interactions. The output module is configured to perform at least one of the following (i) to (iii) in either the currently-presented image or a subsequently-presented image: (i) change the hierarchical order of the image annotation tools in response to the region of interest from which user input is currently received; (ii) display the image annotation complexity metric associated with each region of interest for the medical image that is currently presented; (iii) identify a portion of a region of interest having the most significant impact on the accuracy of the annotation for the region of interest from which user input is currently received. The delineation of the coronary plaque may include identifying an image region which represents the coronary plaque.

Embodiments of the present disclosure pertain to an image annotation method for use in delineating coronary plaque in medical images. The method comprises presenting a medical image and a plurality of image annotation tools having a hierarchical order, the medical image including a plurality of regions of interest. The method further comprises receiving user input corresponding to one or more interactions with each image annotation tool for each region of interest of the medical image. The method further comprises recording each interaction with each image annotation tool for each region of interest of the medical image. The method further comprises computing an image annotation complexity metric for each region of interest of the medical image, based on the recorded plurality of interactions. The method further comprises performing at least one of the following in either the currently-presented image or a subsequently-presented image: (i) changing the hierarchical order of the image annotation tools in response to the region of interest from which user input is currently received; (ii) displaying the image annotation complexity metric associated with each region of interest for the medical image that is currently presented; (iii) identifying a portion of a region of interest having the most significant impact on the accuracy of the annotation for the region of interest from which user input is currently received.

Embodiments of the present disclosure relate to a computer program product comprising instructions which wen executed on a computer cause the computer to carry out the method described in the previous paragraph.

### BRIEF DESCRIPTION OF THE DRAWINGS

Figure 1 is a schematic illustration of a system according to a first exemplary embodiment;
Figure 2A is a schematic illustration of a medical image acquired using an image acquisition system of the system illustrated in Figure 1 wherein the medical image is used for analyzing coronary plaque using the system according to the first exemplary embodiment, which is shown in Figure 1;
Figure 2B is a schematic illustration of regions of interest obtained by annotating the medical image, which is shown in Figure 2A, using the system according to the first exemplary embodiment, which is illustrated in Figure 1;
Figure 3A is a schematic illustration of a graphical user interface of the system according to the first exemplary embodiment, which is shown in Figure 1, during identification of the lumen border;
Figure 3B is a schematic illustration of the graphical user interface, which is shown in Figure 3A, during identification of the media-adventitia border; and
Figure 4 is a schematic illustration of a graphical user interface of a system according to a second exemplary embodiment; and
Figure 5 is a flowchart schematically illustrating a method of analysis of medical images according to an exemplary embodiment.

### DETAILED DESCRIPTION OF EMBODIMENTS

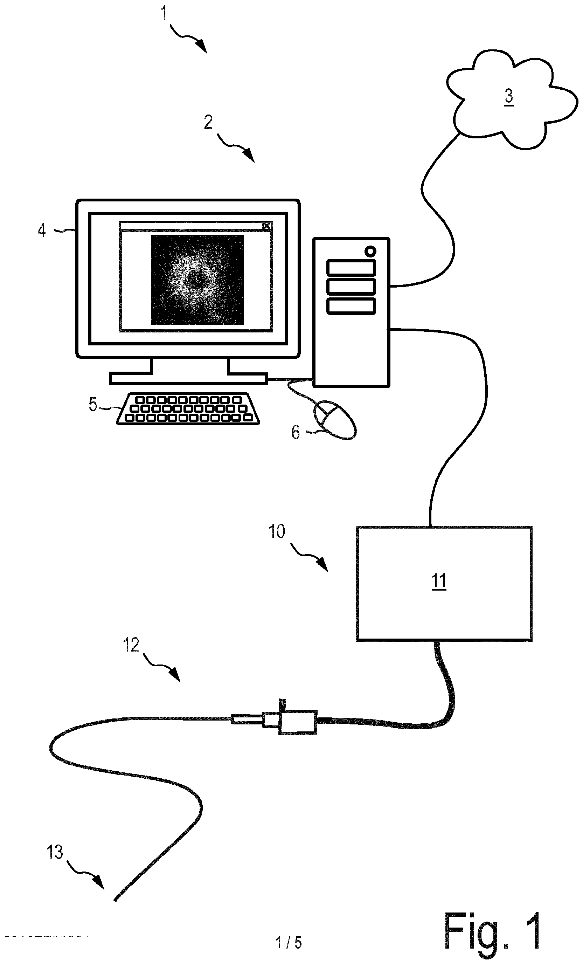

Figure 1 is a schematic illustration of a system 1 according to a first exemplary embodiment. The system 1 includes a medical image annotation system 2 for analyzing two- and/ or three-dimensional medical images. The image annotation system 2 is configured as a data processing system which may be a stand-alone computer and/ or a distributed computer system, which is configured to use a computer network 3, such as the Internet or a local area network, LAN. The image annotation system 2 includes a display device 4, and one or more input devices, such as a keyboard 5 and a computer mouse 6 allowing user interaction via a user interface of the image annotation system 2. The user interface of the exemplary image annotation system 2 is configured as a graphical user interface.

The image annotation system 2 is configured to read and/ or generate medical images that are generated using an image acquisition system 10. In the exemplary embodiment, which is illustrated in Figure 1, the image acquisition system 10 is an intravascular ultrasound, commonly abbreviated as IVUS, system. However, the present disclosure is not limited to IVUS systems, but can be applied to any system, which is configured to acquire two- and/ or three-dimensional medical images from the body. Such systems may are configured to perform one or a combination of the following imaging techniques: angiography, such as coronary CT angiography, abbreviated as cCTA, angioscopy, thermography, fluorescence microscopy, optical coherence tomography (OCT), computer tomography (CT), magnetic resonance imaging (MRI), positron emission tomography (PET), and single-photon emission computed tomography (SPECT).

In the present exemplary embodiment, the medical images are cross-sectional images of a blood vessel and are used to identify plaque, in particular plaque in a coronary blood vessel. Coronary plaque may lead to decreased blood flow in the coronary vessels such that part of the heart muscle is unable to function properly or even dies. Particularly dangerous is vulnerable plaque which has a high likelihood to disrupt. Rupture and subsequent thrombosis of vulnerable plaque is the main cause of Acute Coronary Syndrome (ACS). Specifically, ACS is frequently caused by plaque rupture of nonobstructive, eccentric coronary plaques, which initiates a thrombotic cascade leading to total or near total occlusion of the coronary lumen. Identification of vulnerable plaque using medical images is therefore important to enable the development of treatment modalities to stabilize such plaque.

Plaques having a large necrotic core and a thin fibrous cap are suspected to be rupture prone and are therefore frequently referred to as thin-cap fibroatheroma (TCFA). It has been shown that there is an inverse relationship between plaque cap thickness and risk of plaque rupture. As such, morphological features of the plaque are associated with rupture.

However, it is to be noted that the present disclosure is not limited to image annotations for morphological characterization of plaque, in particular its identification within the image. In general, it is conceivable to use the system for annotation of other body portions. By way of example, the systems and methods described herein can be used in other intravascular treatment procedures, such as peripheral below the knee (BTK) and/ or above the knee (ATK) procedures. Further, the systems and methods described herein can be used for analysis of medical images acquired from vascular stents. Additionally or alternatively, the systems and methods described herein can be used for analysis of medical images acquired from organs and/ or body portions other than blood vessels, such as two- and/ or three-dimensional thorax images. The thorax images may be acquired using computed tomography, projected X-ray imaging and/ or magnetic resonance tomography (MRT).

As the image data of the medical images are two-dimensional and/ or three-dimensional and may have high resolution, it has been shown that the annotation process needs to be at least partly automated since a fully manual annotation at the pixel/voxel level is tedious and time-consuming and therefore does not fit within a clinical workflow which has to deal with an ever-increasing amount of patient data. Manual segmentation also leads to errors and variation in the results, depending intra-operator and inter-operator variabilities. On the other hand, it has been shown that automatic annotation algorithms (i.e. algorithms which do not require user interaction and which may be learned from previously recorded human annotations) do not provide the required accuracy.

Therefore, there is a need for semi-automatic annotation tools (i.e. annotation tools which require user interaction), which allow efficient annotation with high accuracy.

The inventors have found that it is possible to meet this need by providing a system having an image annotation system based on semi-automatic annotation tools as described in the present disclosure.

Such semi-automatic annotation tools may be configured so that the image annotation system proposes an estimate for the annotation. The annotation tool may further be configured so that the operator can adapt the annotation estimate, e.g. by accepting, refining, or rejecting the annotation estimate. Additionally or alternatively, the semi-automatic annotation tool may be configured so that the user inputs, via the user interface, an estimate for an annotation, which is later refined by the image annotation system. It is also conceivable that these processes are combined resulting in an iterative refinement process in which the user and the annotation system alternately refine the annotation.

Returning to Figure 1, the IVUS system 10 includes a catheter 12 which includes an IVUS transducer which is mounted to a distal end section 13 of a catheter 12. The catheter 12 can be inserted into a human blood vessel. The catheter 12 is configured so that the IVUS transducer is rotatable. By way of example, the IVUS transducer may be rotatable so that 360-degree ultrasound sweeps can be generated to provide cross-sectional images of the blood vessel. The IVUS system 10 further includes a controller 11, which is configured to control the rotational movement of the IVUS transducer and to control the operation of the IVUS transducer. The controller 11 of the IVUS system receives the ultrasound imaging signal generated by the transducer and transmits the signal, modified or unmodified, to the image annotation system 2.

Further, applications of IVUS, such as integrated backscatter wavelet analysis and virtual histology have allowed IVUS to characterize plaques as lipid, fibrous tissue, calcification, or necrotic core with high accuracy. Moving the distal end section 13 of the catheter 12 along the axis of the blood vessel and acquiring rotational sweeps at a plurality of different locations along the blood vessel's axis allows generation of three-dimensional images, which may be analyzed using the annotation system described herein.

As will be discussed in detail further below, the image annotation system 2 is configured to perform identification of the lumen border and/ or the media-adventitia border of the blood vessel. This allows two-dimensional or three-dimensional quantitative analysis of the coronary artery wall and plaque.

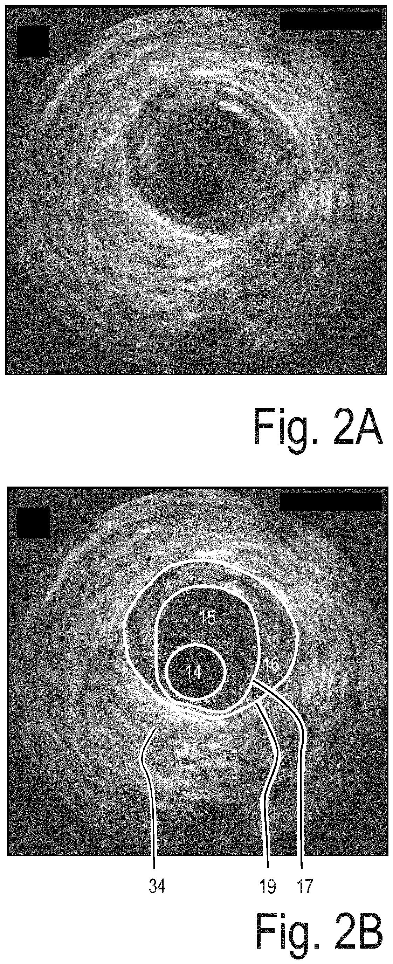

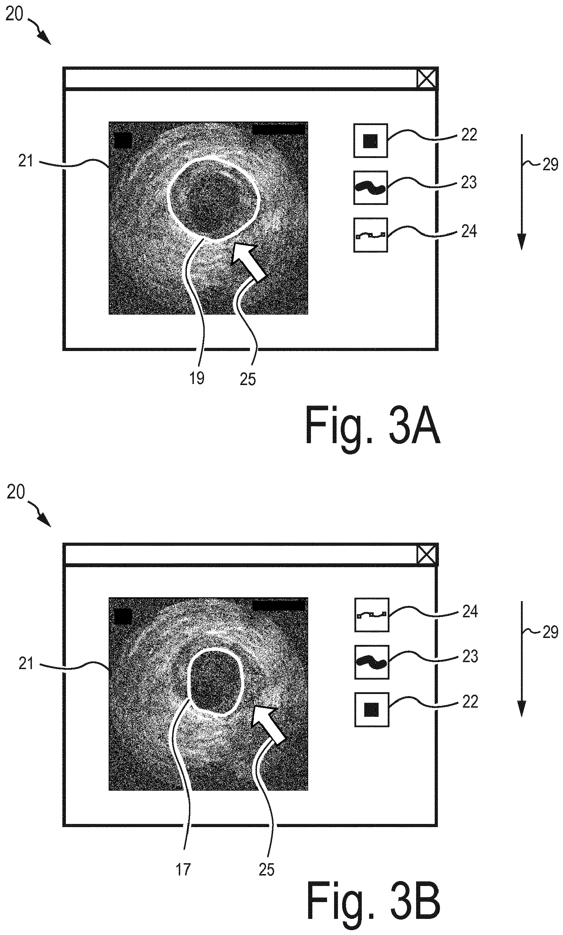

Figure 2A shows a two-dimensional IVUS image, acquired from a coronary blood vessel by performing a 360-degree ultrasound sweep. The image represents a cross-section substantially perpendicular to the blood vessel's longitudinal axis. As is illustrated in Figure 2B, depending on the intensity values of the greyscale image data of the image shown in Figure 2A, it is possible to identify the image region which corresponds to the catheter 14 and the lumen 15 of the blood vessel bounded by the lumen border 17. The lumen 15 is the open channel of the artery through which the blood flows. Furthermore, it is possible to identify the media-adventitia border 19. The adventitia corresponds to an outer covering of the blood vessel. The media represents the wall of the blood vessel and is located in an image region 16 between the lumen border 17 and the media-adventitia border 19. The image region 16 also contains the plaque. The form of the region 16 between the lumen-intima border 17 and the media-adventitia border 19 allows determination whether plaque is present and also a quantitative analysis of the amount of plaque, such as by determining the plaque burden. The further discussion relates to the identification of the lumen border and the media-adventitia border, on which many of the IVUS measurements for plaque analysis rely. However, it is also conceivable that further interfaces and/ or borders are identified, for example, in order to identify the extent of the media and the plaque and/ or to distinguish between calcified plaque and fibro-fatty plaque. Figure 3A is a screenshot of the display device 4 (shown in Figure 1) of the image annotation system 2, which illustrates a window 34 of the graphical user interface, in which at least a portion of the medical image 21 is presented to the user. The graphical user interface is configured to present to the user a plurality of image annotation tools 22, 23 and 24, each of which including one or more operations used in the process for annotating one or more regions of interest of the medical image 21. The annotation tool 22 is a level set / flood fill annotation tool, which performs a flood fill operation depending on a threshold value set by the user and depending on a user-defined starting point for the flood fill operation. The annotation tool 23 is a brush stroke annotation tool, which allows the user to adapt, using brush strokes, an image region identified by the annotation system and/ or identified by the user by defining pixels and/ or voxels which are to be located within the image region and/ or pixels and/ or voxels which are to be located outside the image region. The image region may represent the region of interest to be annotated or may be surrounded by the region of interest to be annotated (such as surrounded by the lumen border or the media-adventitia border). The annotation tool 23 is a Bezier curve annotation tool which allows the user to generate and/ or adapt a region of interest using a Bezier curve. In particular, the Bezier curve annotation tool 23 may be configured to allow the user to adapt a location of one or more control points on a viewing surface of the display device. The region of interest to be annotated may be represented by the Bezier curve or may be surrounded by the Bezier curve.

Each of the annotation tools 22, 23 and 24 is presented to the user by the graphical user interface 20 using an icon. The graphical user interface 20 is further configured to allow the user to select, using the pointer 25 of the computer mouse 6 (shown in Figure 1), one of the annotation tools 22, 23 and 24 which are presented to the user in order to use the selected annotation tool for identifying a region of interest (i.e. selecting the pixels and/ or voxels which form the region of interest).

As is shown in Figure 3A, the annotation tools 22, 23 and 24 are presented to the user in an order, which is schematically indicated in Figure 3A by an arrow 29. The arrow 29 is not displayed by the graphical user interface. The order of the annotation tools 22, 23 and 24 represent, for each of the annotation tools 22, 23 and 24, a rank among the presented annotation tools. The medical image annotation system is configured so that the rank is a measure of efficiency of the image annotation process when the respective image annotation tool is used.

It is also conceivable that other graphical representations are used to present the annotation tools 22, 23 ad 24 to the user in an order. By way of example, the user interface may present to the user a list of items, wherein each of the items represents one of the annotations tools. The rank of an annotation tool in the list may be indicative of the rank of the respective annotation tool among the annotation tools presented using the list.

The image annotation system 2 is configured so that the order of the annotation tools 22, 23 and 24 depends on a region of interest of the medical image displayed using the graphical representation 21, which is currently receiving user input via an input device of the annotation system. By way of example, as is shown in Figure 3A, the user starts to adapt an estimate of the media-adventitia border 19 of the blood vessel which was determined by the annotation system, wherein the user moves the pointer 25 of the computer mouse to the media-adventitia border 19 and clicks a button of the computer mouse. In response thereto, the annotation system arranges the annotation tools 22, 23 and 24 in an order so at that the annotation tool 22, which is the level set/ flood fill annotation tool has a higher rank than the annotation tool 23, which is the brush stroke annotation tool. The brush stroke annotation tool 23, in turn, has a higher rank than the Bezier curve annotation tool 24. The user interface may be configured so that the user can select one or more of the presented annotation tools irrespective of the order in which they are presented, for example, by moving the pointer 25 of the computer mouse to the icon of the annotation tool which is to be selected and by clicking the mouse button of the computer mouse.

Measurements which were acquired by the annotation system from user input based on previously annotated images have shown that the media-adventitia border 19 can be identified by the annotation system with a low degree of user interaction without decreasing the accuracy below a predetermined threshold value. Therefore, an annotation tool, such as the Bezier curve annotation tool 24, which requires a high degree of user interaction, is not necessary for determining the media-adventitia border. Rather, annotation tools, such as the level set / flood fill annotation tool 22 and the brush stroke annotation tool 23 can be used, which allow efficient annotation of the media-adventitia border with a low degree of user interaction.

Figure 3B illustrates the graphical user interface in a state in which the user adapts an estimate for the lumen border 17 which was determined by the annotation system. As is illustrated in Figure 3B, when the user starts to adapt the estimate for the lumen border 17 by moving the pointer 25 of the computer mouse to the lumen border 17 and by clicking the mouse button, the annotation system rearranges the representations of the annotation tools 22, 23 and 24 so that the Bezier curve annotation tool 24 has a higher rank than the brush stroke annotation tool 23 and the brush stroke annotation tool 23, in turn, has a higher rank than the level set / flood fill annotation tool 22. Thereby, the system indicates to the user that for identifying the lumen border, it is more efficient to use the Bezier curve annotation tool 24 than the brush stroke annotation tool 23 or the level set / flood fill annotation tool 22. If the user is more familiar with the brush stroke annotation tool 23 or the bounding box annotation tool, then, the user can still select these annotation tools for annotating the media-adventitia border in the graphic recitation 21 of the medical image. However, the order of the annotation tools indicates to the user that these annotation tools are less efficient and/ or less accurate for performing the annotation task. The user can select the Bezier annotation tool 24 by moving the pointer 25 of the computer mouse to the corresponding icon and by clicking with the mouse button. Then, the lumen border is transformed in to a Bezier curve having control points which can be manipulated by the user, for example, by using the pointer 25 of the computer mouse.

Therefore, adapting the order of the annotation tools to the region of interest from which user input is currently received, allows to guide the user to use the annotation tools in a manner so that a time-efficient annotation of the medical image can be performed.

For each of the regions of interest (i.e. for each of the lumen border and the media-adventitia border), the annotation system determines the order of the annotation tools by computing, for each of the regions of interest, an image annotation complexity metric. The image annotation complexity metric may include one or more parameters. One or more of the parameters may be indicative of an amount or a degree of user interaction required by the user to perform an annotation of the respective region of interest.

By way of example, the annotation system may assign a low parameter value used as image annotation complexity metric to the media-adventitia border (designated with reference numeral 19 in Figure 2B) of the vessel, since the media-adventitia border can easily be recognized by the annotation system in an automatic manner so that simple and easy to handle annotation tools (such as the set level / flood fill annotation tool), which require only a low degree of user interaction, can be used to identify the media-adventitia border so that a sufficiently high accuracy is obtained.

On the other hand, the image annotation system may assign a comparatively high parameter value, which is used as image annotation complexity metric, to the region of interest, which corresponds to the lumen border (designated with reference numeral 17 in Figure 2B), since this border can be determined by the annotation system in an automatic manner only with a low level of accuracy, so that a comparatively high degree of user interaction is required to refine the lumen border calculated by the annotation system in order to obtain a satisfactory level of accuracy.

The image annotation system is configured so that for each of the regions of interest, the order 29 of the annotation tools for the respective region of interest is determined depending on the image complexity metric of the respective region of interest. Therefore, as is illustrated in Figure 3A, if the user starts to annotate the media-adventitia border 19, the annotation system recommends through the order of annotation tools, an annotation tool, which allows the user to finely adjust the media-adventitia border calculated by the annotation system so that the required accuracy can be achieved through a greater amount of user interaction.

For each of the regions of interest, the image annotation complexity metric is determined depending on one or more recorded user interactions which were recorded for the respective region of interest. The interactions may be interactions for the image for which the image annotation complexity metric is determined. Additionally or alternatively, the interactions may be interactions for the same region of interest but made for one or more other medical images.

By way of example, the image annotation system may be configured to determine the image annotation complexity metric depending on a measured time required by the user to annotate at least a portion of the respective region of interest via the user interface.

Additionally or alternatively, the image annotation complexity metric may be determined depending on a number of interactions required by the user to annotate at least a portion of the region of interest via the user interface. The number of interactions may be a measure on the amount of user interaction which is required for the user to obtain the desired accuracy.

Additionally or alternatively, the image annotation complexity metric may be determined depending on which of the plurality of annotation tools are used by the user to annotate at least a portion of the respective region of interest. By way of example, if the user prefers an annotation tool which requires much user interaction for annotating the region of interest, this indicates that annotation of the region of interest requires a high amount of user interaction. The annotation system therefore assigns to this region of interest a parameter value of the image annotation complexity metric which represents a high degree of required user interaction.

Additionally or alternatively, the image annotation complexity metric is determined depending on a number and/ or a geometrical arrangement of the pixels of at least a portion of the respective region of interest. By way of example, the pixels of a region of interest may have a low degree of clusterization. A low degree of clusterization may be present, if the region of interest includes a high number of mutually isolated pixel clusters, each of which having a comparatively small number of pixels. If a region of interest has a high degree of clusterization, the image annotation system may assign to the region of interest an image annotation complexity metric which indicates a high degree of required user interaction.

The image annotation system is further configured to adapt, for one or more of the annotation tools 22, 23 and 24 and for one or more of the regions of interest, operation parameters of the respective annotation tools, depending on measurements acquired from user input representing an interaction using one or more of the annotation tools to annotate the respective region of interest.

By way of example, a number of interactions, which are required by the user to annotate at least a portion of a region of interest via the user interface may be measured. Additionally or alternatively, the image annotation system may measure a time required by the user to perform a predefined annotation task, such as at the annotation of the whole or at least a predefined portion of the region of interest. Additionally or alternatively, the image annotation system may determine how many times a user interacts with a pixel or voxel. By way of example, the annotation system detects that the user repeatedly flips a pixel and/ or voxel of a region of interest. This may be an indication of a particular challenging or important image region. In response to this measurement, the annotation system may adjust parameters of one or more of the annotation tools so as to allow a fine adjustment of the region of interest when the annotation tool is used. By way of example, in response to the detected repeated flips of pixels and/ or voxels, the annotation system may adjust the line width of the brush stroke tool, allowing the user to perform a finer adjustment of the region of interest.

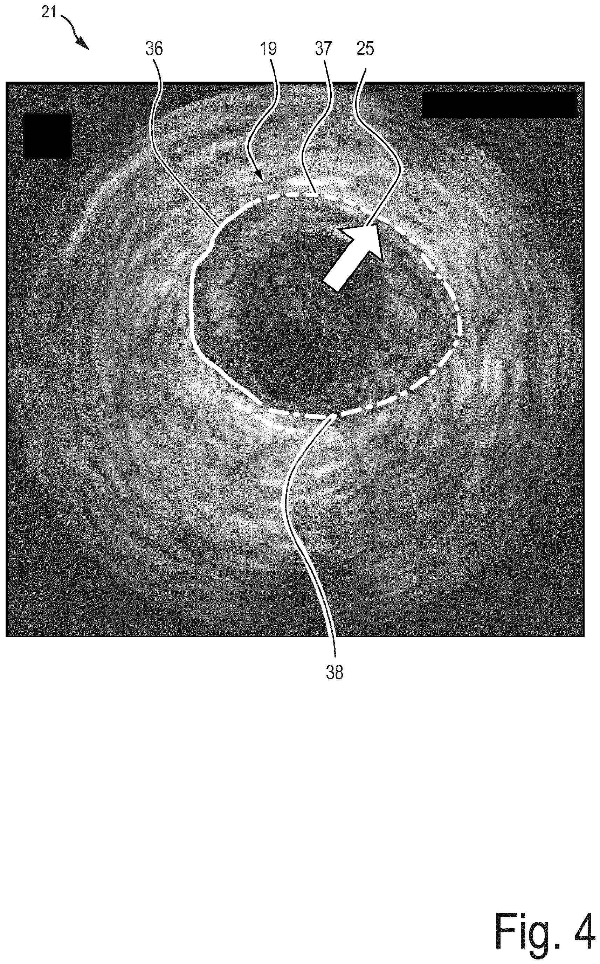

Figure 4 schematically illustrates a graphical user interface of an image annotation system according to a second exemplary embodiment. The image annotation system according to the second exemplary embodiment may include at least a portion of the features of the first exemplary image annotation system, as explained above with reference to Figures 1 to 3B.

In the image annotation system according to the second exemplary embodiment, the image annotation system is configured display, for one or more regions of interest, overlaid on at least a portion of the medical image 21, an indicator, which is indicative of the image annotation metric of the respective region of interest.

As is shown in Figure 4, in the image annotation system according to the second exemplary embodiment, the media-adventitia border 19 includes three regions of interest, wherein for each of these regions of interest, the image annotation system has calculated a separate image annotation complexity metric. It is also conceivable that the media-adventitia border is represented by only one region of interest.

For each of these regions of interest, the annotation system displays a visually perceptible graphical indicator 36, 37 and 38, wherein each of the indicators is indicative of the image annotation complexity metric of the respective region of interest. Specifically, the indicator 38 in the form of a dash-dotted curve indicates a region of interest having an image annotation complexity metric which represents a high degree of required user interaction. The indicator 37 in the form of a dashed curve indicates a region of interest having an image complexity metric which represents a medium degree of required user interaction. The indicator 36 in the form of a solid curve indicates a region of interest having an image complexity metric which represents a low degree of required user interaction.

The indicators 36, 37 and 38 are overlaid over at least a portion of the medical image 21 and also indicate the extent of the respective region of interest.

Accordingly, in the image annotation system of the second exemplary embodiment, the user's attention can be directed to those portions of the image, which require a high amount of user interaction. This allows the user to select for the region of interest, the appropriate annotation tool for annotating the respective region of interest. By way of example, the user may recognize from the indicator in the form of the dash-dotted curve 38 that this region of interest has an image annotation complexity metric which represents a high degree of required user interaction. Therefore, for adapting the region of interest which is represented by curve 38, the user is guided to select an annotation tool, such as the Bezier curve tool, which allows to finely adjust the region of interest via a high degree of user interaction.

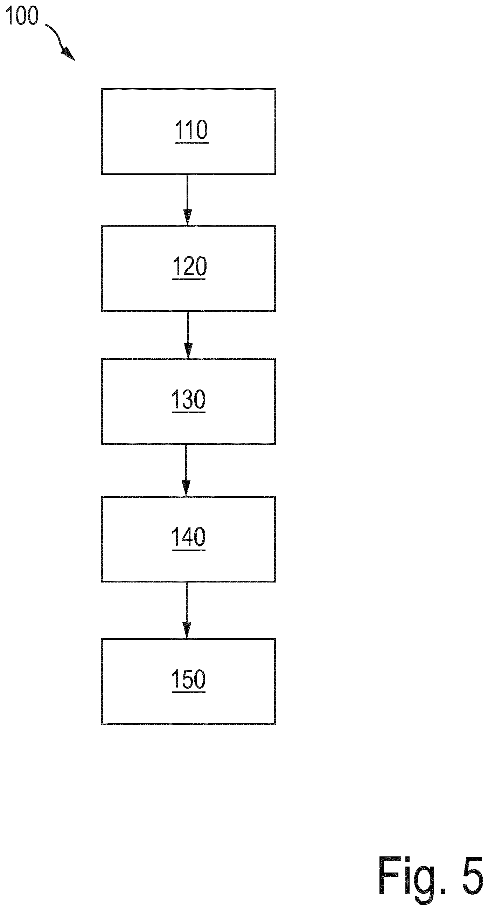

Figure 5 is a flow chart of an exemplary method for annotating medical images which is performed using an image annotation system. The image annotation system presents (110) the medical image and the plurality of image annotation tools to the user using the user interface. Then, the image annotation system receives (120), for each of the regions of interest, user input corresponding to one or more interactions with one or more of the image annotation tools. For the presented image and/ or for one or more images which were previously presented to the user, user interactions are recorded (130) for each of the regions of interest. The previously presented images have the same regions of interest as the image which is currently presented to the user but at different locations within the image.

An image annotation complexity metric (140) is computed for each of the regions of interest, depending on the recorded plurality of interactions. Additionally or alternatively to the computation of the image annotation complexity metric, the annotation tools are presented (150) by the user interface so that the presentation is indicative of an order (29), wherein the order (29) is changed in response to the region of interest from which the user input is currently received. The user input may be received via one or more input devices of the image annotation system, such as a computer mouse or a keyboard.

It is proposed to use a predictive user model trained on the backlog of previous user interaction sessions in order to allow the annotation system to optimize its internal parameters to minimize the interaction time required for the skilled user. This may include one or more of the following features:
- The annotation system may perform experimental design internally i.e. it can vary internal parameters and see whether this has the effect of reducing user annotation effort or not. This may be executed in an online fashion.
- The user may, e.g. via GUI interaction, inform the system how tedious, a certain interaction was perceived. This may allow to incorporate an up-weighting of actions which are subjectively of higher priority and need to influence the optimization criterion accordingly.

The annotation system may thus adapt its parameters. The user may experience a shift in abilities:
- At the beginning, a freshman user may start off by annotating images with brushstrokes or voxel-level annotation and correction tools that act very locally with a small influence region and hence require a lot of user interaction (as measured by interaction time per pixel-to-be-annotated).
- Later when, the user gets more experienced, the user might use higher-level tools such as big brush strokes or region growing tools.

The predictive model may maintain a sensitivity estimate of a number of possible interactions i.e. by estimating the number of annotation voxels flipped when a particular interaction was performed as opposed to a different interaction.
- Repeated flipping in a user interaction session may indicate a particularly challenging and/ or important region.
- This sensitivity measure could be used to guide the user attention by presenting the tools to a user in a particular order or arrangement or by identifying e.g. by highlighting the most relevant or influential voxels by e.g. a heatmap so as to communicate which interaction would have the biggest effect and would hence transmit most information from user to the system.

Using the actual user interaction as training data may jointly improve both the user forward model as well as the segmentation algorithm since both of them are preferably coupled by the objective of minimal user interaction.

Any of the method steps disclosed herein may be recorded in the form of instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present disclosure can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

The above embodiments as described are only illustrative, and not intended to limit the technique approaches of the present disclosure. Although the present disclosure is described in details referring to the preferable embodiments, those skilled in the art will understand that the technique approaches of the present disclosure can be modified or equally displaced without departing from the protective scope of the claims of the present disclosure. In the claims, the word "comprising" does not exclude other elements or steps, and the indefinite article "a" or "an" does not exclude a plurality. Any reference signs in the claims should not be construed as limiting the scope.

## Claims

1. A system (1) comprising a medical image annotation system (2) for analyzing a plurality of two- and/ or three-dimensional medical images, the medical image annotation system providing a plurality of image annotation tools, each of which being configured to perform, for one or more regions of interest of the medical images, at least a portion of an annotation;
wherein the medical image annotation system (2) comprises:
a user interface;
a recording module, and
a computation module,
wherein, for each of the images, the user interface is configured to i) present the respective medical image, and to ii) receive, for each of the regions of interest of the respective image, user input corresponding to one or more interactions using one or more of the image annotation tools (22, 23, 24);
wherein for a first one of the medical images, the recording module is configured to record, for one or more of the regions of interest of the first image, the interactions;
wherein at least one of (a) and (b) applies for the first image and/ or a second image of the images:
(a) the computation module is configured to compute an image annotation complexity metric for each of the regions of interest of the respective image, depending on the recorded interactions; and
(b) a presentation of the annotation tools (22, 23, 24) by the user interface is indicative of an order (29), wherein the order is changed in response to the region of interest of the respective image from which the user input is currently received.

2. The system (1) of claim 1, wherein at least (a) applies and for at least one of the regions of interest of the respective image, the image annotation complexity metric of the region of interest is determined depending on one or more of the interactions recorded for the region of interest.

3. The system (1) of claim 1 or 2, wherein at least (a) applies and for at least one of the regions of interest of the respective image, the image annotation complexity metric is indicative of a degree of user interaction required to annotate at least a portion of the region of interest using the user interface.

4. The system (1) of any one of the preceding claims, wherein at least (a) applies and for at least one of the regions of interest of the respective image, the image annotation complexity metric is determined depending on a measured time required by the user to annotate at least a portion of the region of interest via the user interface.

5. The system (1) of any one of the preceding claims, wherein at least (a) applies and for at least one of the regions of interest of the respective image, the image annotation complexity metric is determined depending on a number of interactions required by the user to annotate at least a portion of the region of interest via the user interface.

6. The system of any one of the preceding claims, wherein at least (a) applies and for at least one of the regions of interest of the respective image, the image annotation complexity metric is determined depending on which of the plurality of annotation tools (22, 23, 24) are used by the user to annotate at least a portion of the region of interest.

7. The system (1) of any one of the preceding claims, wherein at least (a) applies and for at least one of the regions of interest of the respective image, the image annotation complexity metric is determined depending on a number and/ or a geometrical arrangement of the pixels of at least a portion of the region of interest.

8. The system (1) of any one of the preceding claims, wherein (a) and (b) applies and the image annotation system is configured to determine, for at least one of the regions of interest of the respective image, the order of the image annotation tools depending on the image annotation complexity metric of the region of interest.

9. The system (1) of any one of the preceding claims, wherein the user interface is configured to adapt one or more operation parameters of one or more of the image annotation tools depending on one or more of the recorded interactions.

10. The system (1) of claim 9, wherein the system is configured to vary the at least one of the operational parameters; and to measure, how the variation influences measurements acquired from at least a portion of the interactions of the user.

11. The system (1) of any one of the preceding claims, wherein at least (a) applies and the user interface is configured to display, for at least one of the regions of interest of the respective image, an indicator (36, 37, 38) which is visually indicative of the image annotation complexity metric of the region of interest and which is displayed concurrently with the region of interest.

12. The system of any one of the preceding claims, wherein at least (b) applies and the image annotation system is configured to generate a user profile depending on the user input received via the user interface, wherein the order of the annotation tools is determined depending on the user profile.

13. A method (100) of analyzing a plurality of two- and/ or three-dimensional medical images using an image annotation system (2) having a user interface and providing a plurality of image annotation tools, each of which being configured to perform, for one or more regions of interest of the medical images, at least a portion of an annotation;
wherein the method comprises for each of the images:
presenting (110), using the user interface, the respective medical image;
receiving (120), using the user interface, for each of the regions of interest of the respective image, user input corresponding to one or more interactions using one or more of the image annotation tools; and
wherein the method comprises for a first one of the images: recording (130), using the image annotation system (2), for each of the regions of interest of the first image, the interactions; and
wherein at least one of (a) and (b) applies for the first image and/ or a second image of the images:
(a) the method (100) further comprises computing (140) an image annotation complexity metric for one or more of the regions of interest of the respective image, depending on the recorded interactions; and
(b) the method (100) further comprises presenting (150), using the user interface, the annotation tools so that the presentation is indicative of an order (29), wherein the order (29) is changed in response to the region of interest of the respective image from which the user input is currently received.

14. Computer program product comprising instructions which wen executed on a computer cause the computer to carry out the method (100) of claim 13.

15. A program element for analyzing a plurality of two-dimensional or three-dimensional medical images using an image annotation system (2) having a user interface, wherein the medical image annotation system provides a plurality of image annotation tools, each of which being configured to perform, for one or more regions of interest of the medical image, at least a portion of an annotation;
wherein the program element, when being executed by a processor of the data processing system, is adapted to carry out for each of the images:
presenting (110), using the user interface, the respective medical image;
receiving (120), using the user interface, for each of the regions of interest of the respective image, user input corresponding to one or more interactions using one or more of the image annotation tools;
wherein the program element, when being executed by a processor of the data processing system, is adapted to carry out for a first one of the images:
recording (130), using the image annotation system (2), for one or more of the regions of interest of the first image, the interactions; and
wherein at least one of (a) and (b) applies for the first image and/ or a second image of the images:
(a) the program element, when being executed by a processor of the data processing system, is adapted to carry out: computing (140) an image annotation complexity metric for each of the regions of interest of the respective image depending on the recorded interactions; and
(b) the program element, when being executed by a processor of the data processing system, is adapted to carry out: presenting (150) the annotation tools, using the user interface, so that the presentation is indicative of an order (29), wherein the order (29) is changed in response to the region of interest of the respective image from which the user input is currently received.
